# EUROPEAN PATENT APPLICATION

(11) **EP 2 149 340 A1**
(43) Date of publication of application: **03.02.2010**
(21) Application number: 08761579.5
(22) Date of filing: 09.05.2008
(51) Int. Cl.: A61B 17/3213

(54) **BISTOURY COMPRISING A BLADE EJECTOR**

(30) Priority: 10.05.2007 ES 200701356
(71) Applicant: Corell Alcantarilla, Rafael, 46020 Valencia (ES)
(72) Inventor: MARTINEZ CASAÑ, Pedro Arturo, E-46020 Valencia (ES)
(74) Representative: Chanza Jordan, Dionisio
(86) International application number: PCT/ES2008/000334
(87) International publication number: WO 2008/139012

(57) **Abstract**

The invention relates to a bistoury comprising a blade ejector for reducing the risk of cuts or injury to medical personnel, said bistoury comprising an ejector shaft (2) received inside the central part of the body of a bistoury (1) assembled by means of guides (3) and rods (4) allowing the horizontal movement of the ejector shaft in order to trigger the release of a knife (5) by various ejecting mechanisms and movements, including application of pressure and horizontal movement, horizontal movement via a ramp element arranged on the head, horizontal movement via a ramp element arranged on the ejector, and horizontal movement with a fold-down tab.

## Description

### Technical sector

The invention protected by this Patent consists in a bistoury with a disposable blade ejection mechanism to be used in medical and surgical activities.

As such, it is a bistoury that includes an ejecting pin for the secure ejection of the blade.

### Previous technique

Since distant past, the medical and veterinary industries have employed surgical instruments with fixed blades or razors that must be sterilized before each use, or, more recently, instruments with disposable blades that must be replaced during these medical procedures, thus placing the physical integrity of its users at risk on account of unintentional and accidental injuries.

The interest in minimising risks of accidental injury for medical personnel has already been made evident with previous invention developments such as European Patents n° 2195380 (T3) RAPHAEL MOSSERI about a Protection device for cutters (1998) trought a hinge, and the Spanish Utility Models No. ES171103 MEDICO HOSPITALARIA,S.A. related to a protective device for scapels (1971) with a hood for the blade, or N° ES158871 CELESTINO PEREZ GRANEL related to a hood or wing as blade protector (1970), and the Utility Models n° ES295795 BECTON, DICKINSON AND COMPANY related to a mechanism to fix the cutting blade into the scapel (1085) and the n° ES292081 by the same applicant.

### Technical Problem

Consequently, known systems focus either on the protection of the edge of the razor or blade, or on the interchangeable and disposable character of the blade, however they both show limitations since it is necessary for the users to exert manual pressure with their fingers directly on the blade in order to replace it. For bistouries with a blade permanently fixed to the handle, a sterilization of the blade before each use is required, which adds to the costs, time and medical supervision, while for bistouries containing interchangeable single-use blades, no method has been developed which permits to eject rapidly and immediately the used blade without direct intervention with the fingers. Further, with a single handle, multiple blades of various shapes and functions may also be used: for cutting and removing sutures. This is the aim of the invention being presented herein.

### Technical solution

Thus, with the new invention the removal of the used blade will be achieved by an ejection mechanism located in the handle of the bistoury itself which allows to remove it without handling it directly, offering a consequent reduction in risks of cuts or lesions. And this as a simplified bistoury with a handle comprised of two parts: a handle-head, and a handle-ejecting pin.

### Advantages

To this aim, the present invention will introduce a solution allowing to reduce risks of cuts for medical personnel when the blades have to be changed or at the moment of finalisation of the medical intervention which may lead to both muscular lesions in the hand tendons, as well as the transmission of viral or bacteriological infecting agents through the wounds sustained.

Further, these frequent occurrences in the medical and veterinary profession, as they are being reduced with the invention being patented promote the policy of prevention of work-related accidents in the health profession.

### Description of the figures

For a better understanding of the general characteristics mentioned above, several drawings are attached to the present invention and illustrate their specifications as follows:

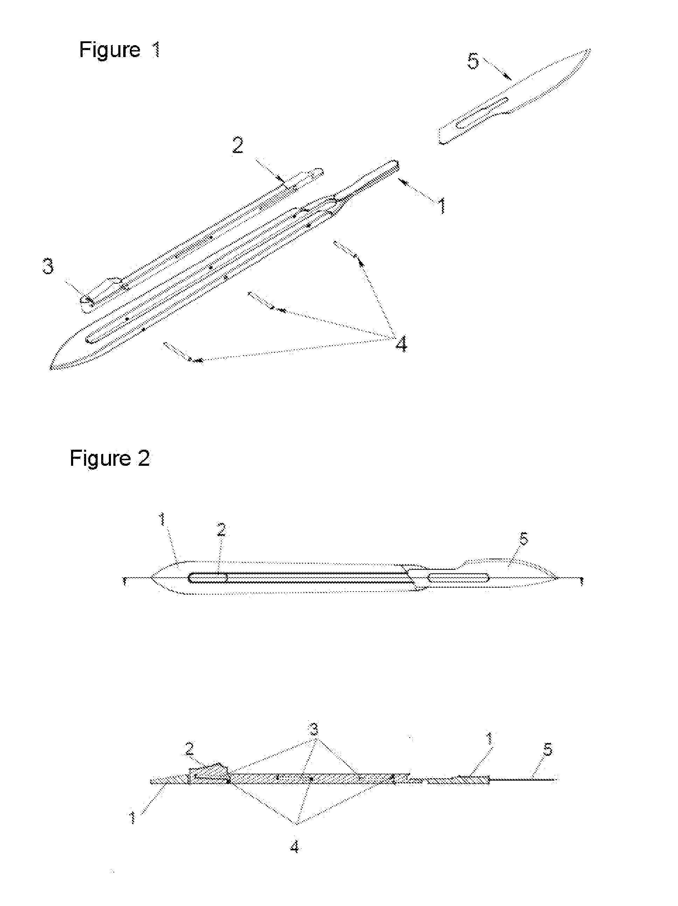

**Figure 1****:** Projection of an exploded view of the body of the bistoury with its handle-head **(1)** and its blade **(5)**, with inclusion of a handle-ejecting pin **(2)** in the central section of its body with several guiding grooves **(3)** through which several guiding rods **(4)** are assembled on the ejecting pin.
**Figure 2****:** Profile view and lateral section view of the bistoury illustrating, in position of operation, the handle-head **(1),** the handle-ejecting pin **(2)** with the guiding grooves **(3),** guiding rods **(4)**, and the blade **(5).**

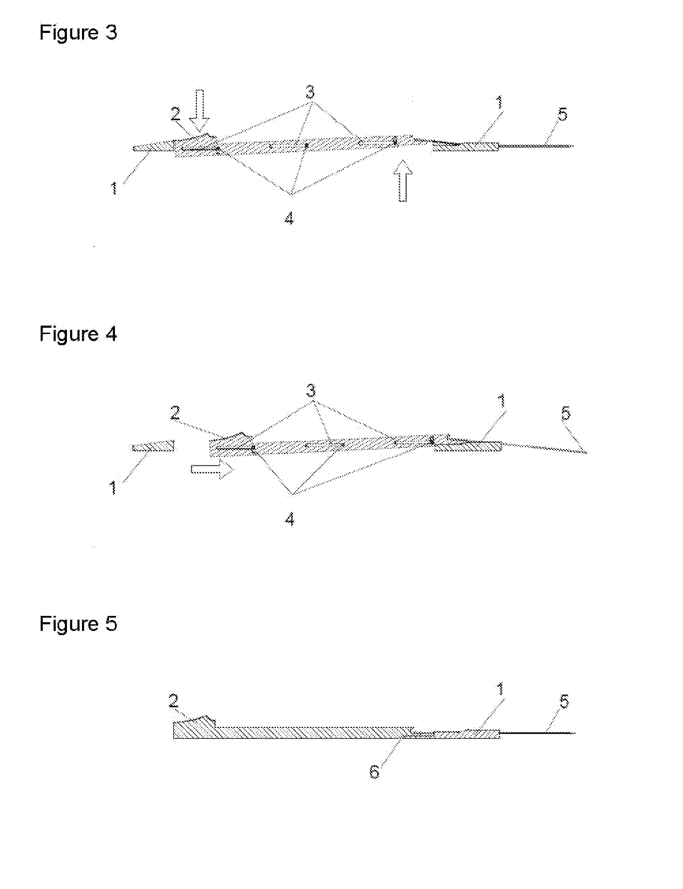

**Figure 3****:** Lateral section view of the bistoury illustrating how, when pressure is exerted on its posterior section, the handle-ejecting pin **(2)** swings onto the guiding grooves **(3)** and guiding rods **(4)**, removing the blade **(5)** thanks to its right angle-shaped finish upon which the blade rests.
**Figure 4****:** Lateral section view of the bistoury illustrating how, when it rests on the handle-head **(1),** the handle-ejecting pin **(2)** pushes and moves the blade **(5)** horizontally along the guiding grooves **(3)** and guiding rods **(4)**, releasing it completely from the head of the bistoury.
**Figure 5****:** Lateral section view of the bistoury illustrating the handle-head of the bistoury **(1)** one extremity of which has a finish in shape of a ramp **(6)** that allows it to connect with the handle-ejecting pin **(2)** which after being moved horizontally, rises and disengages the blade **(5)** and releases and expels it completely from the head. With a detailed view of the superposition and coupling of the ramp of the handle-head with the handle-ejecting pin.

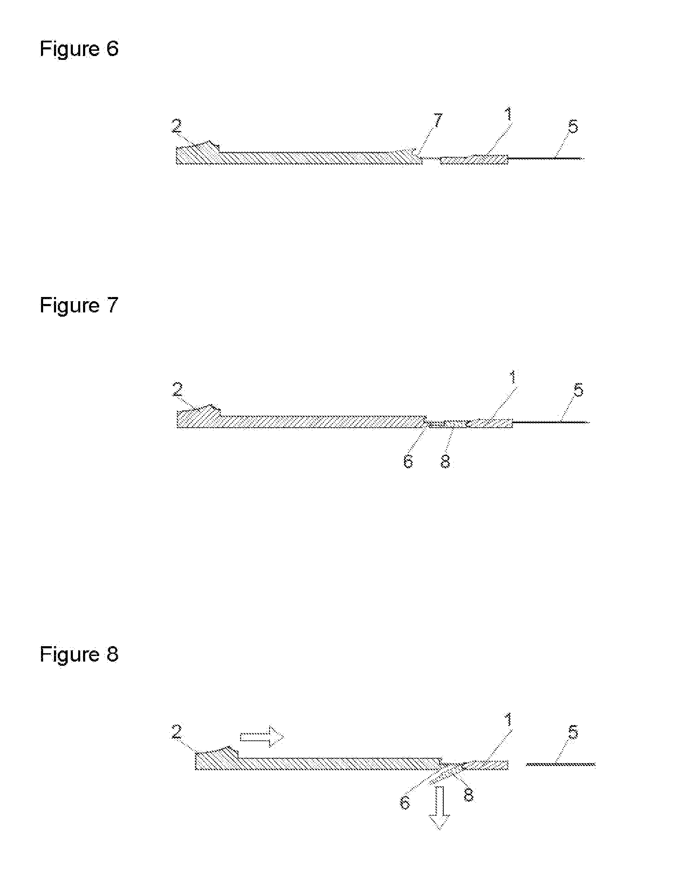

**Figure 6****:** Lateral section view of the bistoury illustrating the handle-ejecting pin **(2)** one extremity of which has a finish in shape of a ramp **(7),** and onto which the blade **(5)** rests and is supported, resting securely on the handle-head **(1)**.
**Figure 7****:** Lateral section views of the bistoury in position of operation and at the moment of removal and release of the blade respectively, illustrating that the handle-ejecting pin **(2)** has one extremity in the shape of a straight ramp **(9)** which, when it is moved horizontally, allows the folding flap **(8)** to be disengaged from the head of the bistoury's body **(1)**, which then releases the blade **(5).**

### Mode of implementation of the invention

With reference to the invention described herein, depending on the configuration of the ejecting pin or the body of the bistoury itself, particularly that of its handle-head, various ejecting modes of the blade may be possible. In all cases, the handle-ejecting pin will be lodged within the body of the bistoury and will be assembled to it by several guiding rods that insert into the guiding grooves of the pin. In this form, pressure and/or horizontal movement of the pin allows the blade to be removed and ejected safely through the different variations described herein.

We can define three main types of configurations or implementations of the bistoury depending on the performance of the ejecting mechanism:
A first type of **expulsion mechanism by swinging and horizontal movement,** when the extremity of the handle-ejecting pin in contact with the blade may have a right-angle finish on which the blade rests. With it, by applying pressure on its extremity, the handle-ejecting pin **(2)** swings onto the guiding grooves **(3)** and raises and lifts one end of the blade **(5)** thanks to its right angle-shaped finish. Thus, after pushing horizontally the ejecting pin **(2),** the latter moves the blade **(5)**, removing it completely.
A second type **of expulsion mechanism by horizontal movement via ramp** with two variants. One of them, the ramp-head variant, where the handle-head of the bistoury **(1)** can have a ramp-shaped finish **(6)** who allows the handle-ejecting pin **(2)** to travel upwards when it is moved horizontally, thus disengaging the blade **(5)** and releasing completely from the head of the handle **(1)**. And, conversely, the ramp-ejecting pin variant, where the extremity of the handle-ejecting pin **(2)** in contact with the blade can have a ramp-shaped finish **(7)** which, when it is moved horizontally, allows the blade **(5)** to be disengaged and then completely ejected from the head.

And finally, a third type of **expulsion mechanism by horizontal movement with folding flap** in which the handle-head of the bistoury **(1)** can include a folding flap **(8)** which, when it makes contact with the ramp **(9)** of the handle-ejecting pin **(2)** and is pushed horizontally, lowers and actions the flap, releasing the blade **(5)** in order to eject it.

## Claims

1. Bistoury with blade ejection mechanism **characterised in that** it includes a handle-ejecting pin (2) lodged within the central part of a handle-head (1) assembled through several guiding grooves (3) and several guiding rods (4) who allow the ejecting pin to travel horizontally in order to move a blade (5).

2. Bistoury with blade ejection mechanism complying with Claim No.1 **characterised in that** the extremity of the handle-ejecting pin **(2)** in contact with the blade has a right-angle finish on which the blade rests. With it, by applying pressure on its back end, the handle-ejecting pin swings and pivots onto the guiding grooves **(3)** supported by several guiding rods placed transversally **(4),** and with its front end lifts the blade **(5)** thanks to its right angle-shape mounted in the front part of the handle-head. Thus, after having been released and pushed horizontally, the ejecting pin **(2)** moves the blade **(5)** and ejects it completely.

3. Bistoury with blade ejection mechanism complying with Claim No.1 **characterised in that** an internal extremity of the handle-head **(1)** is shaped as a ramp, allowing the ejecting pin **(2)**, when moved horizontally, to connect and raise, and whose movement disengages the blade **(5)** mounted in the front part of the handle-head **(1),** releasing and ejecting it through its very movement.

4. Bistoury with blade ejection mechanism complying with Claim No.1 **characterised in that** the handle-head **(1)** has a folding flap **(8)** which, when it makes contact with the ramp **(9)** of the handle-ejecting pin **(2)** and is pushed horizontally, lowers and actions the flap, releasing the blade (5) releasing and ejecting it of the handle-head **(1).**

5. Bistoury with blade ejection mechanism complying with Claim No.1 **characterised in that** the handle-head **(1)** has a folding flap **(8)** which, when it makes contact with the ramp **(9)** the ejecting pin **(2)** when moved horizontally, movement disengages the blade **(5)** to eject.
